# EUROPEAN PATENT SPECIFICATION

(11) **EP 3 160 523 B1**
(45) Date of publication and mention of the grant of the patent: **01.04.2020**
(21) Application number: 15731475.8
(22) Date of filing: 16.06.2015
(51) Int. Cl.: A61L 15/42, A61L 15/46, A61L 15/24

(54) **ANTIMICROBIAL COMPOSITIONS UTILIZING SILVER AND OXYGEN, PROCESS FOR MAKING, AND METHOD OF USING THE SAME**
ANTIMIKROBIELLE ZUSAMMENSETZUNGEN MIT VERWENDUNG VON SILBER UND SAUERSTOFF, VERFAHREN ZUR HERSTELLUNG UND VERFAHREN ZUR VERWENDUNG DAVON
COMPOSITIONS ANTIMICROBIENNES UTILISANT DE L'ARGENT ET DE L'OXYGÈNE, LEURS PROCÉDÉS DE FABRICATION ET D'UTILISATION

(30) Priority: 26.06.2014 US 201462017350 P
(43) Date of publication of application: 03.05.2017
(73) Proprietor: Avent, Inc., Alpharetta, GA 30004 (US)
(72) Inventor: GANN, John P., Alpharetta, Georgia 30004 (US); ZHAO, Zhongju L., Alpharetta, Georgia 30004 (US)
(74) Representative: Dehns
(86) International application number: PCT/US2015/035977
(87) International publication number: WO 2015/200037

(56) References cited:
- US-A1- 2008 306 183
- US-A1- 2012 265 124
- "Differential Effect of Common Ligands and Molecular Oxygen on Antimicrobial Activity of Silver Nanoparticles versus Silver Ions", ENVIRON. SCI. TECHNOL., vol. 45, 2011, pages 9003-9008, XP55193277, cited in the application

## Description

### FIELD OF THE INVENTION

The present invention relates to generally to the field of wound treatment devices for the delivery of gases and other agents to compromised tissues.

### BACKGROUND

Silver is commonly used as a topical antimicrobial agent for chronic and acute wounds. It is incorporated into various formulations such as ointments, hydrogels, and polymers and then applied topically to wounds. Silver is also used in various wound treatment devices. One of the drawbacks with the use of silver is that, at high levels, it can be cytotoxic to healthy mammalian cells, and cause inflammation that will inhibit wound healing.

Ionic silver was found to be cytotoxic to human fibroblasts at a concentration of1 part per million see Kvitek, A, et al, Antibacterial activity and toxicity of silver - nanosilver versus ionic silver. Journal of Physics: Conference Series 304 (2011) 012029. Similar results were found by Hidalgo E., et. al. (1998, Silver Nitrate: antimicrobial activity related to cytotoxicity in cultured human fibroblasts. Skin Pharmacol Appl Skin Physiol) wherein they found silver nitrate to be cytotoxic down to 0.7 parts per million. There are numerous silver containing wound care products that may release much higher levels of silver than this. This cytotoxicity could potentially slow the wound healing process.

As silver is used more and more in both the consumer and wound care arenas; there is the potential for certain organisms to develop tolerances to it at low concentrations due to chronic exposure. These would include especially gram negative bacteria where biocides are prevented from reaching their target in the cell by the outer membrane and active efflux systems. See Poole, K. J., Antimicrob. Chemother, (July 2005) 56 (1): 20-51. The literature shows various examples of bacterial organisms that have a propensity to develop resistance to silver such as Psuedomonas aeruginosa, Salmonella typhimurium, Enterobacter cloacae, Acinetobacter baumannii, and Serratia marcescens. See Lansdown, A., Williams, A., (2007) Bacterial resistance to silver-based antibiotics. Nursing Times; 103: 9, 48-49.

It has been demonstrated that in order for silver to have antimicrobial properties it must be in its ionized (Ag+) form. Silver salts and other silver containing compounds act by releasing small amount of silver ions; typically these ions are released by dissociation of the silver salt. Silver nanoparticles have a slightly different mechanism; the nanoparticles at their core are made up of zero valent silver metal which is insoluble in water, the surface of these particles must first oxidize to form Ag₂O, which then can dissociate to give off silver ions. See S.L. Percivala, P.G. Bowlera, D. Russell, Bacterial resistance to silver in wound care. Journal of Hospital Infection (2005) 60, 1-7 See also Zong-Ming Xiu, Jie Ma, and Pedro J. J. Alvarez, Differential Effect of Common Ligands and Molecular Oxygen on Antimicrobial Activity of Silver Nanoparticles versus Silver Ions; Environ. Sci. Technol. 2011, 45, 9003-9008.

Silver containing compounds with extremely low dissociation constants tend to show very little if any antimicrobial activity since there is such a low level of dissociation of silver ions. This is especially true of silver when it forms complexes with certain halogens, like iodine, or when it forms complexes with molecules that possess functional groups containing sulfur, like thiols or disulfides. One theory of how silver might exert its antimicrobial efficacy is that it binds with thiols found in proteins from bacterial cell walls and internal organelles, disrupting the proteins' tertiary structure and rendering them unable to function.

Wound beds are typically protein rich environments. These proteins can come from the patient's own wound exudate and/or microbial biofilms and typically contain numerous thiol groups and disulfide groups. When these thiols or disulfides interact with silver ions to form silver-sulfide complexes, the silver ions becomes highly insoluble before they can reach the target bacteria and are rendered much less effective as an antimicrobial agent.

Generally speaking, when silver is applied to a wound by way of conventional topical wound dressing much, if not most, of it will very likely be rendered insoluble and thus ineffective by the proteins. Many silver wound products employ some sort of silver "reservoir" in the form a slightly soluble silver salt like silver chloride or in the form elemental silver such as colloids or nanoparticles. It is thought that the silver ions are released in a slow controlled fashion as to deliver an antimicrobial effect over a longer duration of time. In reality it is possible that these low levels of ion silver are simply overwhelmed by the abundance proteins, each of which would contain numerous deactivating thiols and disulfide groups.

While it has been suggested that reactive oxygen species may play a role in silver's antimicrobial activity, the role is not well understood. For example, one study demonstrated that silver ions exhibited better bactericidal activity against *E*. *coli* and *S*. *aureus* under aerobic vs. anaerobic conditions. This activity was attributed to intracellular reactive oxygen species (ROS) generation of the superoxide radical. It was suggested that the mechanism involved the thiol interaction between silver and cellular enzymes. See Hee-Jin Park, Jee Yeon Kim, Jaeeun Kim, Joon-Hee Lee, Ji-Sook Hahn, Man Bock Gu, Jeyong Yoon. Silver-ion-mediated reactive oxygen species generation affecting bactericidal activity. Water Research 43 (2009) 1027-1032. In another study, cells were exposed to silver nanoparticles of varying sizes. It was found that the smaller the nanoparticles the more cytotoxic they were. The cells were found to have high levels of the typical markers associated with oxidative stress suggesting a ROS mechanism. C. Carson, et. al. Unique Cellular Interaction of Silver Nanoparticles: Size Dependent Generation of Reactive Oxygen Species. J. Phys. Chem. B 2008, 112, 13608-13619.

Conversely, it has been shown that ROS are not the only mechanism by which silver has antimicrobial activity. In Differential Effect of Common Ligands and Molecular Oxygen on Antimicrobial Activity of Silver Nanoparticles versus Silver Ions; Environ. Sci. Technol. 2011 , 45, 9003-9008, it was demonstrated that silver nitrate, which completely dissociates in water to give high levels of silver ions, had identical kill rates in both anaerobic and atmospheric aerobic environments. Since molecular oxygen is required to generate ROS, the equivalent kill rates suggest that ROS are not involved with the mechanism. The paper shows that silver nanoparticles show efficacy against E. coli under anaerobic conditions. Exposing the particles to ambient air increased the kill rate presumably by oxidizing the surface of the particles which would increase the silver ion release as described previously.

Other related art includes US2012/0265124 which discloses a biosorbable wound treatment device, process for making, and a method of using the same.

Accordingly, there is a need for a method, composition or device for treating wounds utilizing antimicrobial silver materials without being cytotoxic to healthy mammalian cells, and without causing inflammation that inhibits wound healing. There is also a need for a method, composition or device for treating wounds that utilize antimicrobial silver materials at low concentrations while making it more difficult for certain organisms to readily develop tolerances to silver at low concentrations. There is also a need for a method, composition or device for treating wounds that speeds up the kill rate of antimicrobial silver materials in order to reduce the likelihood of microbial resistance to silver.

These needs extend to methods, devices and compositions that utilize antimicrobial silver materials in protein rich environments such as wound beds, while reliably and efficiently providing satisfactory levels of microbial kill. There is also an unmet need for a method of potentiating antimicrobial silver materials against pathogens. For example, there is an unmet need for treating or preventing microbial infection in a mammal and/or enhancing wound healing, in which antimicrobial silver is used more effectively than in conventional methods, devices and compositions.

### SUMMARY

In response to the difficulties and problems discussed herein, the present invention provides methods, devices and compositions for potentiating antimicrobial silver materials against pathogens.

In one aspect, the present invention provides a wound dressing, as claimed in claim 1.

In another aspect, the present invention provides a method of potentiating antimicrobial silver material against pathogens, as claimed in claim 9.

The present invention may encompass a wound treatment device for potentiating antimicrobial silver materials against pathogens, the device including: a) a formed biocompatible polymeric matrix, wherein the matrix includes closed cells and walls; b) gaseous oxygen, wherein the gaseous oxygen is contained in the closed cells; and c) an antimicrobial silver material present on at least a skin-contacting portion of the matrix. According to the invention, when such a device is used to treat a wound, oxygen is delivered from the closed cells in the presence of the antimicrobial silver material such that the antimicrobial silver material provides greater microbial kill than antimicrobial silver material alone. For example, the antimicrobial silver material may provide at least a 3 log increase in microbial kill than just the antimicrobial silver material alone as measured by antimicrobial challenge assays. For example, the antimicrobial silver material may provide at least a 3 log increase in microbial kill for gram-positive bacteria within three hours of treatment than just the antimicrobial silver material alone as measured by antimicrobial challenge assays. Generally speaking, the antimicrobial silver material may provide at least a 1 log increase in microbial kill for gram-negative bacteria within three hours of treatment than just the antimicrobial silver material alone as measured by antimicrobial challenge assays.

The antimicrobial silver material may be a silver salt, a silver complex, elemental silver, or a combination thereof. For example, the antimicrobial silver may be a silver salt and a silver complex. As another example, the antimicrobial silver may be elemental silver by itself or in combination with a silver salt and/or a silver complex. The elemental silver may be an elemental silver material selected from silver powder and silver nanoparticles.

The antimicrobial silver material may be present in a concentration of from about 0.001 weight percent to less than 0.5 weight percent. For example, the antimicrobial silver material may be present in a concentration of from about 0.0025 weight percent to less than 0.5 weight percent. As another example, the antimicrobial silver material may be present in a concentration of from about 0.005 weight percent to less than 0.5 weight percent. As yet another example, the antimicrobial silver material may be present in a concentration of from about 0.0075 weight percent to about 0.25 weight percent. Desirably, the antimicrobial silver material may be present at a concentration of from about 0.05 weight percent to about 0.0025 weight percent. It is contemplated that the antimicrobial silver material may be present at levels less than 0.0025 weight percent. For example, in some cases the antimicrobial silver material may be present at a level of from about 0.000001 to about 0.001 weight percent.

The biocompatible polymeric matrix may comprise a polymer and a catalyst, and gaseous oxygen is produced when a peroxide is reacted with the catalyst so that dissolved oxygen is present in moisture in the walls. The catalyst used in the wound treatment device may be sodium carbonate. However, other catalysts, for example, salts of alkali metals and alkali earth metals may be used provided they are consistent with the product being biocompatible. In addition, more than one catalyst may be used. For instance, one catalyst may be derived from a group consisting of salts of alkali metals and alkali earth metals and the second catalyst may include, but are not limited to, organic and inorganic chemicals such as cupric chloride, ferric chloride, manganese oxide, sodium iodide and their equivalents. Other catalysts, include, but are not limited to enzymes such as lactoperoxidase and catalase or a combination thereof.

The peroxide used in the wound treatment device is desirably hydrogen peroxide. However, other peroxides, including, but not limited to, ammonium peroxide, urea peroxide, sodium peroxide, and other peroxy compounds or a combination thereof can be used provided they leave no residue that would be inconsistent with biocompatibility and/or bioabsorption. The present invention contemplates use of components that can generate a gaseous element within the matrix and that are safe and effective for use. For example, an acid catalyst can be incorporated in the matrix followed by perfusion of the matrix with a carbonate to generate carbon dioxide gas within the matrix. Such materials are then used to buffer solutions or environments.

The devices of the present invention may take many physical forms, depending on uses of the devices. A preferred shape is a gel sheet that can be cut or molded into any two dimensional shape. Other preferred embodiments are primarily constructed of thin strands of matrix suitable for placement into the wound bed or cavity. It is contemplated that the biocompatible matrix of the device may also be a biosorbable matrix.

The present invention further encompasses a method of potentiating antimicrobial silver material against pathogens. The method includes the steps of: providing an antimicrobial silver material to a surface or article to be treated; providing oxygen at the surface or article at greater than atmospheric concentration in the presence of the antimicrobial silver so the antimicrobial silver material provides greater microbial kill than antimicrobial silver material alone. It is to be understood that such a surface or article to be treated can be an inanimate article or surface. The method encompasses the steps of providing an antimicrobial silver material and providing oxygen at greater than atmospheric concentration to a wound.

The antimicrobial silver material may be elemental silver such as silver nanoparticles. Alternatively and/or additionally, the antimicrobial silver material may be silver salts and/or silver complexes or the like. Desirably, these silver salts and/or silver complexes are present at a concentration of from about 0.001 weight percent to less than 0.5 weight percent. More desirably, the antimicrobial silver material is present in a concentration of from about 0.01 weight percent to less than 0.5 weight percent. For example, the antimicrobial silver material is present in a concentration of from about 0.01 weight percent to about 0.25 weight percent. According to the invention, a portion of the antimicrobial silver material may be present in the wound as silver complexes having very low dissociation constants. The low dissociation constant is used to reduce the cytoxicity issues caused by high concentrations of silver while still providing enough silver ion to be antimicrobial.

The oxygen may be provided from a formed matrix that includes closed cells and walls, wherein gaseous oxygen may be contained in the closed cells and dissolved oxygen may be present in moisture in the walls, and the antimicrobial silver material may be provided from antimicrobial silver material present on a portion of the matrix.

Other objects, advantages and applications of the present disclosure will be made clear by the following detailed description.

### BRIEF DESCRIPTION OF THE DRAWINGS

FIG. 1 is an illustration showing a cross-section an exemplary wound treatment device.
FIG. 2 is an illustration of a graph depicting the log reduction in Pseudomonas aeruginosa CFU on the y-axis and time (hours) on the x-axis for various samples including examples of the present invention.
FIG. 3 is an illustration of a graph depicting the log reduction in Methicillin-resistant Staphylococcus aureus CFU on the y-axis and time (hours) on the x-axis for various samples including examples of the present invention.

### DETAILED DESCRIPTION

Reference will now be made in detail to one or more embodiments, examples of which are illustrated in the drawings. It should be understood that features illustrated or described as part of one embodiment may be used with another embodiment to yield still a further embodiment. It is intended that the claims include these and all other modifications coming within the scope of the appended claims.

As used herein, the term "antimicrobial challenge assays" refers to conventional antimicrobial effectiveness tests and includes tests based on ASTM E-2315 "Guide for Assessment of Antimicrobial Activity Using a Time-Kill Procedure".

As used herein, the terms "antimicrobial silver", "antimicrobial silver materials", "silver" and "silver materials" are used interchangeably and refer to materials that elute silver ions in an appropriate form and sufficient quantity to have a measurable microbial kill as determined by antimicrobial challenge assays. For example, these materials may be silver salts, silver complexes, and elemental silver in the form of silver nanoparticles or silver powder.

As used herein, the term "gram-negative bacteria" refers to bacteria identified utilizing a gram stain technique (also called "Gram's method") in which a violet dye is applied, followed by a decolorizing agent, and then a red dye. The gram-negative bacteria lose the first dye and appear red.

As used herein, the term "gram-positive bacteria" refers to" refers to bacteria identified utilizing a gram stain technique (also called "Gram's method") in which a violet dye is applied, followed by a decolorizing agent, and then a red dye. The gram-positive bacteria retain the first dye and appear violet.

As used herein, the terms "potentiate" and "potentiating" refer to the enhancement of one agent by another so the combined effect is greater than the sum of the effects of each one alone.

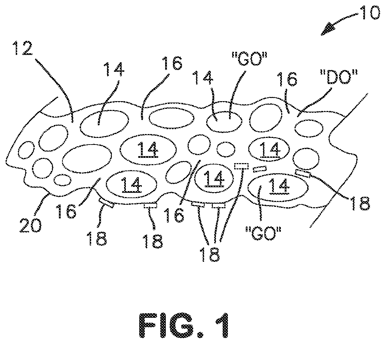

The present invention provides methods, devices and compositions for potentiating antimicrobial silver materials against pathogens. In one aspect, the present invention encompasses a wound treatment device for potentiating antimicrobial silver materials against pathogens. Referring to FIG. 1 , there is illustrated in cross-section a portion of an exemplary wound treatment device 10 (not necessarily to scale). The device includes a formed biocompatible polymeric matrix 12. The biocompatible polymeric matrix 12 is used for the delivery of oxygen. The matrix may include a water swellable, cross-linked biocompatible polymer network. Exemplary biocompatible matrices and techniques to form the same are disclosed by U.S. Patent No. 8,679,523 issued March 25, 2014 to Gibbins et al. for "Oxygen-Delivery Closed Cell Foam Matrix for Wound Treatment" and U.S. Patent No. 7,160,553 issued January 9, 2007 to Gibbins et al. for "Matrix for Oxygen Delivery to Compromised Tissues".

The matrix 12 includes closed cells 14 and walls 16. That is, a plurality of gas-permeable, elastic, closed cells is defined by the cross-linked biocompatible polymer network. According to an aspect of the invention, the biocompatible matrix may be a biocompatible polymeric matrix that includes a polymer and a catalyst and the closed cells may be produced from a reaction between a catalyst and a second reactant (e.g., a peroxide). Deliverable oxygen is contained within the elastic closed cells such that when the device is used to treat a wound, oxygen is delivered from the closed cells. Generally speaking, gaseous oxygen ***"GO"*** is contained in the closed cells 14 and dissolved oxygen ***"DO"*** is present in moisture in the walls 16.

The biocompatible polymer network is formed from a biocompatible polymer that can be cross-linked into a water-swellable polymer network. The cross-linked polymer network should be flexible such that it can define elastic, closed cells that are also gas permeable. A variety of polymers either naturally derived or synthetic may be used to prepare polymer network. Polymers that are cross-linkable and biocompatible are preferred. Exemplary polymers include, but are not limited to, hyaluronic acid and its derivatives, alginic acid and its derivatives, collagen, chitosan, chitin, starch derivatives, gums such guar gum, xanthan gum, citric acid based polymers, lactic acid and glycolic acid based polymers, poly(aspartates), poly(orthoesters), poly (phosphazenes), poly(anhydrides), poly(phosphoesters) and polyalkylene glycol based polymers. Other exemplary polymers include, but are not limited to polylysine, polyacrylamide, polymethacrylate, polyethylene, polyacrylate, polybuterate, polyurethane foam, polyether, silastic, silicone elastomer, rubber, nylon, vinyl or cross-linked dextran. If cross-linked dextran is used, it is preferred that the molecular weight of the dextran polymer is between 50,000 and 500,000. Additionally, the matrix material can be made from a combination of natural and synthetic polymers, or mixtures of synthetic polymers or mixtures of natural polymers.

The second reactant is desirably hydrogen peroxide. However, other peroxides, including, but not limited to, urea peroxide, sodium peroxide and other peroxy compounds can be used provided they leave no residue that would be inconsistent with biocompatibility and/or bioabsorption. The present invention contemplates use of components that can generate a gaseous element within the matrix and that are safe and effective for use. For example, an acid catalyst can be incorporated in the matrix followed by perfusion of the matrix with a carbonate to generate carbon dioxide gas within the matrix. Such materials are then used to buffer solutions or environments.

The catalyst may be sodium carbonate. However, other catalysts such as other alkali and alkali earth compounds may be used provided they are consistent with the product being biocompatible. In addition, more than one catalyst may be used. For instance, one catalyst may be derived from a group consisting of salts of alkali metals and alkali earth metals and the second catalyst may include, but are not limited to, organic and inorganic chemicals such as cupric chloride, ferric chloride, manganese oxide, sodium iodide and their equivalents. Other catalysts, include, but are not limited to enzymes such as lactoperoxidase and catalase.

The biocompatible matrix may include a non-gellable polysaccharide. Examples of suitable non-gellable polysaccharides include guar gum, guar gum, lucerne, fenugreek, honey locust bean gum, white clover bean gum, or carob locust bean gum. The biocompatible matrix may further include a plasticizer. The plasticizers may be glycerol and/or water, however, propylene glycol and/or butanol and combinations thereof may also be used. If glycerol is used, a range of between approximately 0.25 to 25% w/w, preferably between 0.5 to 12% w/w. The biocompatible matrix may further include a hydration control agent. The hydration control agent may be an isopropyl alcohol; however, ethanol, glycerol, butanol, and/or propylene glycol and combinations thereof may also be used. A range of isopropyl alcohol of between approximately 0.05 to 5% w/w, preferably between approximately 0.1 to 2.5% w/w and most preferably between approximately 0.25 to 1% w/w is generally sufficient. For example, when the cross-linked biocompatible polymer network is formed, the degree of water-swelling may be increased by adding a non-gellable polysaccharide such as guar gum and by adding a hydration control agent such as glycerol. By making the cross-linked biocompatible polymer network sufficiently water-swellable, the second reactant (e.g., hydrogen peroxide) may be absorbed into the cross-linked biocompatible polymer network. To assess the swellability of biocompatible matrix by aqueous fluids, it is important to measure its hydration capacity, expressed as "water absorption capacity" at 25 degrees Centigrade after an immersion or soak time in distilled water after 3 to 4 hours. Useful levels of water absorption capacity may range from about 2 to about 8 grams of water per gram of biocompatible polymer network. For example, the level of water absorption capacity may range from about 3 to about 7 grams of water per gram of biocompatible polymer network. As another example, the level of water absorption capacity may range from about 4 to about 6 grams of water per gram of biocompatible polymer network.

It is contemplated that the biocompatible matrix may further include a water loss control agent. To decrease the permeability of the matrix, water loss control agents may be applied to a surface of the device. Application of water loss control agents may be useful since a decrease in the permeability of the device controls the loss of fluids. Suitable water loss control agents include glycolipids, ceramides, free fatty acids, cholesterol, triglycerides, sterylesters, cholesteryl sulfate, linoleic ethyl ester, and silicone oil. Additionally, the compositions and devices may have an impermeable sheet covering one or more surfaces to aid in control of moisture.

Alternatively and/or additionally, it is contemplated that the biocompatible polymeric matrix may be in the form of one or more gas reservoirs having one or more types of gas contained therein such as generally described in U.S. Patent No. 8,075,537 issued December 13, 2011 to Franklin et al. for a "Multiple Cell Therapeutic Diffusion Device".

The gas reservoir may be made of a gas permeable polymeric film or may incorporate portions made of such materials. The gas reservoir may also be in the form of a "bubble wrap" type materials containing oxygen gas wherein at least a portion of the individual cells is permeable to oxygen gas. As another example, the gas reservoir may be in the form of a chamber or bag used to administer hyperbaric treatment gas to a region of the skin as described in U.S. Patent No. 4,224,941 issued September 30, 1980 to Stivala for "Hyperbaric Treatment Apparatus".

One or more antimicrobial silver material(s) 18 is present on at least a skin-contacting portion 20 of the matrix 12. For example, the antimicrobial silver material 18 may be incorporated throughout the matrix 12 as a result of inclusion in the matrix 12 during compounding. Alternatively and/or additionally, the antimicrobial silver material 18 may be applied to the matrix 12 utilizing dipping, coating, deposition processes, spraying processes or similar techniques.

The one or more antimicrobial silver material 18 may be a silver salt, a silver complex, elemental silver, or a combination thereof. For example, the antimicrobial silver may be a silver salt and a silver complex. As another example, the antimicrobial silver may be elemental silver by itself or in combination with a silver salt and/or a silver complex. The elemental silver may be an elemental silver material selected from silver powder and silver nanoparticles. Exemplary antimicrobial silver nanoparticle materials are disclosed in U.S. Patent No. 8,361,553 issued January 29, 2013 to Karandikar et al. for "Methods and Compositions for Metal Nanoparticle Treated Surfaces". The antimicrobial silver material 18 is not necessarily shown to scale and may be a thin film coating that is residue of a silver salt or silver complex sol or solution and/or may be particles including nano-scale particles (i.e., nanoparticles).

The antimicrobial silver material may be present in a concentration of from about 0.001 weight percent to less than 0.5 weight percent. For example, the antimicrobial silver material may be present in a concentration of from about 0.0025 weight percent to less than 0.5 weight percent. As another example, the antimicrobial silver material may be present in a concentration of from about 0.005 weight percent to less than 0.5 weight percent. As yet another example, the antimicrobial silver material may be present in a concentration of from about 0.0075 weight percent to about 0.25 weight percent. Generally speaking, for wound treatment devices the antimicrobial silver material may be present in relatively low concentrations such as from about 0.025 to about 0.05 weight percent. It is believed that the present invention potentiates antimicrobial silver materials when such materials are present at levels less than 0.0025 or even 0.001 weight percent. For example, the antimicrobial silver materials may be present at levels from about 0.000001 to about 0.001 weight percent.

The devices of the present invention may take many physical forms, depending on uses of the devices. These devices may be left in place and then removed. If the devices are biosorbable, they may be resorbed by the body, instead of being removed. A preferred shape is a gel sheet that can be cut or molded into any two dimensional shape. Other preferred embodiments are primarily constructed of thin strands of matrix suitable for placement into the wound bed or cavity. The devices may be placed in their entirety into a wound, placed in combination with additional bundles of the same design into the wound, or cut through the bridge between strands to reduce the size or number of strands present in the wound. Exemplary structures include, but are not limited to, those described in U.S. Patent No. 5,928,174 for "Wound Dressing Device" issued July 27, 1999 to Gibbins.

According to the invention, when the device is used to treat a wound, oxygen is delivered from the closed cells in the presence of the antimicrobial silver material such that the antimicrobial silver material provides greater microbial kill than antimicrobial silver material alone. For example, the antimicrobial silver material may provide at least a 3 log increase in microbial kill than just the antimicrobial silver material alone as measured by antimicrobial challenge assays. For example, the antimicrobial silver material may provide at least a 3 log increase in microbial kill for gram-positive bacteria within three hours of treatment than just the antimicrobial silver material alone as measured by antimicrobial challenge assays. Generally speaking, the antimicrobial silver material may provide at least a 1 log increase in microbial kill for gram-negative bacteria within three hours of treatment than just the antimicrobial silver material alone as measured by antimicrobial challenge assays.

Generally speaking, when oxygen is supplied for the purposes of wound healing, it is known to help with some metabolic processes that rely on oxygen during wound healing. However, oxygen by itself is not known to be antimicrobial except in the case of certain types of anaerobic bacteria (obligate anaerobes) and is not typically understood as lowering bio-burden in the wound.

While references such as, for example, U.S. Patent No. 7,160,553 issued January 9, 2007 to Gibbins et al. for "Matrix For Oxygen Delivery To Compromised Tissues" and U.S. Patent No. 8,679,523 issued Mary 25, 2014 to Gibbins et al. for "Oxygen-Delivery Closed Cell Foam Matrix For Wound Treatment", propose adding or incorporating various active agents (including silver salts and elemental silver) in certain types of devices used to delivery oxygen to wounds, these references fail to recognize that silver in the presence of dissolved oxygen is more potent against pathogens than just silver alone.

According to the present invention, silver and molecular oxygen provides a synergistic effect on both aerobic (*Psuedomonas aeruginosa*) and anaerobic (*Methicillin-resistant Staphylococcus aureus* - *MRSA)* bacteria. While it might not be entirely surprising to observe that oxygen and silver may be more effective against an obligate anaerobe than silver alone, the inventors believe that the boost in efficacy against a facultative anaerobe (e.g., *MRSA*), which does metabolize oxygen, provided by the combination of oxygen and silver is surprising and unexpected. Similarly it also surprising and unexpected to see the same increase in efficacy provided by oxygen and silver against a fully aerobic organism like *Psuedomonas aeruginosa.* Moreover, It can also be shown that silver is more effective at lower concentrations in the presence of dissolved oxygen than just silver alone.

In order for silver to have antimicrobial properties it must be in its ionized (Ag+) form. Silver containing compounds with extremely low dissociation constants tend to show very little if any antimicrobial activity since there is such a low level of dissociation of silver ions. This is especially true of silver when it forms complexes with molecules that possess functional groups containing sulfur, like thiols or disulfides. It is believed that the antimicrobial efficacy of silver may be related to silver binding with thiols found in proteins from bacterial cell walls and internal organelles. This is thought to disrupt the proteins' tertiary structure and renders them unable to function. Many of the proteins found in wounds come from the patient's own wound exudate and/or microbial biofilms. These contain thiols or disulfides that diminish the antimicrobial efficacy of silver by forming insoluble silver-sulfide complexes before silver ions can reach their target bacteria. It is possible that the presence of oxygen helps to maintain the efficacy of silver by keeping it in the oxidized +1 state in presence of proteins until it can ingress into the bacteria where it can render its antimicrobial effect. Another possible theory is that the microbes themselves can metabolize the silver thiol complexes in the presence of oxygen. Without being bound to any particular theory, the inventors have found that the presence of oxygen (e.g., oxygen topically available at a wound or tissue surface) in combination with antimicrobial silver materials in the wound environment appears to mitigate this effect and increase the antimicrobial effectiveness of the silver in the wound environment. This increase in antimicrobial effectiveness of the silver in the wound environment is thought to take place even in spite of reduced silver ion concentrations.

The present invention further encompasses a method of potentiating antimicrobial silver material against pathogens. The method includes the steps of: providing an antimicrobial silver material to a surface or article to be treated; providing oxygen at the surface or article at greater than atmospheric concentration in the presence of the antimicrobial silver so the antimicrobial silver material provides greater microbial kill than antimicrobial silver material alone. In some embodiments, the surface can be the skin or a wound. In additional embodiments, the article or surface can be an inanimate surface (i.e., a surface that is not the surface of a human or animal body). Any suitable inanimate article or surface can be treated, such as a medical device; a piece of medical equipment; industrial or household surfaces such as countertops, shelves, etc.; consumer products; etc. The method can further encompass providing an antimicrobial silver material and providing oxygen at greater than atmospheric concentration to a wound. Generally speaking, the antimicrobial silver materials may be silver nanoparticles or silver powder. Alternatively and/or additionally, the antimicrobial silver materials may be silver salts or silver complexes.

In carrying out the method of the present invention, the concentration of these antimicrobial silver materials may be present in a concentration of from about 0.001 weight percent to less than 0.5 weight percent, but desirably are present at concentrations of less than 0.5 weight percent. For example, the antimicrobial silver material may be present in a concentration of from about 0.0025 weight percent to less than 0.5 weight percent. As another example, the antimicrobial silver material may be present in a concentration of from about 0.005 weight percent to about 0.4 weight percent. As yet another example, the antimicrobial silver material may be present in a concentration of from about 0.0075 weight percent to about 0.25 weight percent. Generally speaking, when carrying out the method of the present invention in connection with wounds, the antimicrobial silver material may be present in relatively low concentrations such as from about 0.025 to about 0.05 weight percent. It is believed that the present invention potentiates antimicrobial silver materials when such materials are present at levels less than 0.0025 or even 0.001 weight percent. For example, the antimicrobial silver materials may be present at levels from about 0.000001 to about 0.001 weight percent.

According to an aspect of the method invention, the oxygen may be provided from a formed matrix that includes closed cells and walls, wherein gaseous oxygen is contained in the closed cells and dissolved oxygen is present in moisture in the walls, and the antimicrobial silver material may be provided from antimicrobial silver material present on a portion of the matrix.

The present invention also encompasses a wound dressing that includes an oxygen delivery component and an antimicrobial silver material, such that when the wound dressing is used to treat a wound, oxygen is delivered in the presence of the antimicrobial silver material so the antimicrobial silver material provides greater microbial kill than antimicrobial silver material alone. The antimicrobial silver material may be silver nanoparticles and/or silver power. Alternatively and/or additionally, the antimicrobial silver material may be silver salts and/or silver complexes. While embodiments not in accordance with the present invention may utilize higher concentrations of antimicrobial silver material (e.g., up to 2 weight percent, up to 1 weight percent), it is believed that the potentiating presence of oxygen allows the antimicrobial silver material to be present and effective in relatively low concentrations such as from about 0.025 to less than 0.05 weight percent. It is believed that the present invention potentiates antimicrobial silver materials when such materials are present at levels less than 0.0025 or even 0.001 weight percent.
For example, the antimicrobial silver materials may be present at levels from about 0.000001 to about 0.001 weight percent.

An exemplary method of treating or preventing microbial infection in a mammal which is not encompassed by the claimed invention administering an antimicrobial silver material to a site to be treated and providing oxygen at the site at greater than atmospheric concentration in the presence of the antimicrobial silver so the antimicrobial silver material provides greater microbial kill than antimicrobial silver material alone. Another exemplary method comprises treating a bacterial infection by administering an antimicrobial silver material and providing oxygen at greater than atmospheric concentration in the presence of the antimicrobial silver so the antimicrobial silver material provides greater microbial kill than antimicrobial silver material alone. Another exemplary method comprises enhancing wound healing by administering an antimicrobial silver material to a wound and providing oxygen to the wound at greater than atmospheric concentration in the presence of the antimicrobial silver so the antimicrobial silver material provides greater microbial kill than antimicrobial silver material alone.

In the practice of these methods, the antimicrobial silver material may be silver nanoparticles and/or silver power. Alternatively and/or additionally, the antimicrobial silver material may be silver salts and/or silver complexes. While embodiments not in accordance with the present invention, may utilize higher concentrations of antimicrobial silver material (e.g., up to 2 weight percent, up to 1 weight percent), it is believed that the potentiating presence of oxygen allows the antimicrobial silver material to be effectively administered in relatively low concentrations such as from about 0.025 to less than 0.05 weight percent. It is believed that the present invention potentiates antimicrobial silver materials when such materials are administered at levels less than 0.0025 or even 0.001 weight percent. For example, the antimicrobial silver materials may be administered at levels from about 0.000001 to about 0.001 weight percent.

In the practice of these methods, the antimicrobial silver and the oxygen may be provided utilizing the above-described matrix structures and/or the compositions.

An exemplary process, not encompassed by the claimed invention, for making a wound treatment device for potentiating antimicrobial silver materials against pathogens will now be described. The process generally involves at least the steps of: providing a gelling mixture of at least one cross-linkable biocompatible polymer and a catalyst; cross-linking the biocompatible polymer of the gelling mixture to form a water swellable, cross-linked biocompatible polymer network; drying the gelling mixture to a gel sheet; adding a second reactant to the gel sheet; and generating a plurality of closed cells (i.e., closed cells and walls) containing oxygen in the gel sheet by reacting the catalyst and the second reactant. The antimicrobial silver material may be added to the mixture during compounding (e.g., added to the gelling mixture) or may be applied after the gel sheet is formed or after the plurality of closed cells containing oxygen is formed in the gel sheet. The antimicrobial silver material may be added to the gel sheet by dipping, coating, spraying or similar processes. Alternatively and/or additionally, the antimicrobial silver can be incorporated directly into small cavities of the formed matrix of the wound treatment device by spraying or coating or by incorporation during the polymerization of the matrix. It is thought that the release of the antimicrobial silver ions may be controlled by manipulation of antimicrobial silver concentration, movement of water through the matrix, and the degree of cross linking in the matrix. In some instances, the release of the antimicrobial silver ions may be controlled by the erosion rate of the matrix *in vivo.*

Generally speaking, the process for making an exemplary wound treatment device of the present invention generates a biocompatible matrix for delivering oxygen and for providing antimicrobial silver. A feature of the biocompatible matrix is the formation of the foam or array of bubbles that entrap the gas. The foam or bubbles are formed by the permeation of the second reactant added to the formed matrix that includes a reactant. When the two reactants interact, a reaction occurs that liberates gas which is entrapped within the matrix. For example, a matrix has a carbonate catalyst (a reactant) incorporated within it. The formed matrix is then placed in the presence of the second reactant (e.g., a peroxide compound such as, for example, hydrogen peroxide). A catalytic decomposition of hydrogen peroxide occurs resulting in the liberation of oxygen gas which becomes entrapped as bubbles formed in situ. The hydrogen peroxide reactant is not part of the compounding of the matrix, but it is in the treatment after the formation of the matrix stock.

According to the process, a gelling mixture of at least one cross-linkable biocompatible polymer is prepared. This may be accomplished by creating a solution of a cross-linkable biocompatible polymer or a solution composed of a mixture of cross-linkable biocompatible polymers. The solution is desirably an aqueous solution or a solution where water component is the major component. According to the process, antimicrobial silver material may be added to the gelling mixture. For example, silver salts, silver complexes, silver nanoparticles and/or silver powder may be added to the gelling mixture.

A catalyst may be introduced into the gelling mixture. For example, the catalyst may be sodium carbonate. However, other catalysts such as other alkali and alkali earth compounds may be used provided they are consistent with the product being biocompatible. In addition, more than one catalyst may be used. For instance, one catalyst may be derived from a group consisting of salts of alkali metals and alkali earth metals and the second catalyst may include, but are not limited to, organic and inorganic chemicals such as cupric chloride, ferric chloride, manganese oxide, sodium iodide and their equivalents. Other catalysts, include, but are not limited to enzymes such as lactoperoxidase and catalase. Desirably, the catalyst is a material that interacts with the second reactant.

A non-gellable polysaccharide, plasticizer and/or a hydration control agent may be added to the gelling mixture. Desirably, the non-gellable polysaccharide is a non-gellable galactomannan macromolecule such a guar gum. A concentration range of guar gum between approximately 0.005 to 53% w/w, preferably between approximately 0.05 to 5% w/w, and most preferably between approximately 0.25 to 1% w/w is generally sufficient. Examples of other suitable non-gellable polysaccharides include lucerne, fenugreek, honey locust bean gum, white clover bean gum, or carob locust bean gum.

The plasticizer(s) may be glycerol and/or water, however, propylene glycol and/or butanol and combinations thereof may also be used. If glycerol is used, a range of between approximately 0.25 to 25% w/w, preferably between 0.5 to 12% w/w, and most preferably between approximately 2.5 to 8% w/w is generally sufficient. The biocompatible matrix may further include a hydration control agent.

The hydration control agent may be an isopropyl alcohol; however, ethanol, glycerol, butanol, and/or propylene glycol and combinations thereof may also be used. A range of isopropyl alcohol of between approximately 0.05 to 5% w/w, preferably between approximately 0.1 to 2.5% w/w and most preferably between approximately 0.25 to 1% w/w is generally sufficient.

The cross-linkable biocompatible polymer of the gelling mixture is then cross-linked to form a water swellable, cross-linked biocompatible polymer network. This may be accomplished by activating a cross-linking agent already present in the gelling mixture, adding or applying a cross-linking agent to the gelling mixture, dehydrating or removing solvent from the gelling mixture, and/or otherwise creating conditions in the gelling mixture that causes cross-linking (e.g., pH, heat, various forms of radiation including electromagnetic radiation and x-rays, ultrasonic energy, microwave energy and the like).

For example, the gelling mixture may be cross-linked by activating a cross-linking agent and dehydrating the gelling mixture to a flexible substrate (i.e., a gel sheet) that can be readily handled. The gel sheet may range in thickness from a few millimeters to 20 millimeters or more. As another example, the gelling mixture may be cross-linked by pouring into a flat open container to maximize surface area and placing the open contain in a conventional oven at an elevated temperature (e.g., 45 to 65°C) and dehydrating for 2 to 6 hours until the sheet reaches a consistency similar to "fruit leather" or "fruit roll-up". Fruit leather or fruit roll-up has a gravimetric or weight-based moisture content of about 10 to about 20 percent. Desirably, the matrix is flexible and elastic, and may be a semi-solid scaffold that is permeable to substances such as aqueous fluids, silver salts, and dissolved gaseous agents including oxygen. Though not wishing to be bound by any particular theory, it is thought that the substances permeate the matrix through movement via intermolecular spaces among the cross-linked polymer. Antimicrobial silver material may be added to the gel sheet at this point in the process. For example, silver salts, silver complexes, silver nanoparticles and/or silver powder may be added by spraying, dipping or coating.

A second reactant is then added to the gel sheet. This may be accomplished by immersing the gel sheet in the second reactant, or by spraying, brushing, coating or applying the second reactant. Desirably, the second reactant is hydrogen peroxide. The hydrogen peroxide may be from 5% wt. to 20% or more. Other peroxides may be substituted, including, but not limited to, urea peroxide, sodium peroxide or other peroxy compounds of alkali metal or alkali earth metals. The second reactant preferably is present in aqueous solution or a solution wherein water is major component.

The second reactant is absorbed into the gel sheet and permeates the swellable, (e.g., water-swellable) cross-linked biocompatible polymer matrix. The degree of swelling may be increased by adding a non-gellable polysaccharide such as guar gum and by adding a hydration control agent such as glycerol. By making the cross-linked biocompatible polymer network sufficiently water-swellable, the second reactant (e.g., hydrogen peroxide) may be adequately absorbed into the cross-linked biocompatible polymer network.

When the second reactant is absorbed and permeates into the cross-linked biocompatible polymer matrix, a gas is generated when the catalyst (i.e., the first reactant) reacts with the second reactant. The resulting gas is desirably oxygen gas. The matrix of the present invention forms a foam or array of bubbles that entrap the gas. That is, a plurality of closed cells containing oxygen is generated in the gel sheet by reaction between the catalyst (i.e., the first reactant) and the second reactant.

For example, a cross-linked biocompatible polymer matrix may have a carbonate catalyst (i.e., a first reactant) incorporated within it. The cross-linked biocompatible polymer matrix is then placed in the presence of the second reactant, hydrogen peroxide. A catalytic decomposition of hydrogen peroxide occurs resulting in the liberation of oxygen gas which becomes entrapped as bubbles formed in situ. The hydrogen peroxide reactant is not part of the compounding of the matrix, but it is added after the formation of the matrix stock.

In finished form the gel sheet transforms into a foam sheet. Antimicrobial silver material may be added to the gel sheet at this point in the process. For example, silver salts, silver complexes, silver nanoparticles and/or silver powder may be added by spraying, dipping or coating.

It is contemplated that the process may further include the step of heating or adding energy to the gel sheet and second reactant to speed up the reaction or to enhance incorporating of antimicrobial silver material. As noted above, one or more antimicrobial silver materials (e.g., silver salts, silver complexes, silver nanoparticles and/or silver powder) may be incorporated at any suitable step in the method. For example, antimicrobial silver materials may be added to the gelling mixture prior to cross-linking, antimicrobial silver materials may be added to the cross-linked polymer matrix prior to dehydration or after dehydration, and/or antimicrobial silver materials may be added to the cross-linked polymer matrix after the closed cells are formed.

The present invention is further described by the examples which follow.

### EXAMPLES

All chemicals used in the examples described below were reagent grade unless specified otherwise.

### Example 1 - Oxygen-Containing Foam Coated With Silver Nanoparticles

A polymer matrix containing oxygen gas was produced as an exemplary wound treatment device to deliver oxygen and antimicrobial silver material. The device was a closed cell foam sheet with oxygen gas contained in the cells of the foam. The closed cell foam sheet was made in accordance with the process described in the Detailed Description above and U.S. Patent No. 7,160,553 issued January 9, 2007 to Gibbins et al. for "Matrix For Oxygen Delivery To Compromised Tissues" and U.S. Patent No. 8,679,523 issued Mary 25, 2014 to Gibbins et al. for "Oxygen-Delivery Closed Cell Foam Matrix For Wound Treatment". The ingredients and their concentrations are listed in Table 1 below.

After formation, the closed cell foam sheet was sprayed or dip coated with a silver nanoparticle solution in a non-polar organic solvent to impart a coating of silver nanoparticles on the surface of the sheet. In use, the wound treatment device would be placed on a wound where it would then release the oxygen into the wound fluid. The silver would also be released such that the combination of silver and oxygen would yield the synergistic benefit described in the previous section.

| **TABLE 1** | | | |
|---|---|---|---|
| **OXYGEN-CONTAINING FOAM COATED WITH SILVER NANOPARTICLES** | | | |
| **Ingredient** | **Pre-Drying Concentration (Weight Percent of Batch)** | **Post-Drying Concentration (Weight Percent of Batch)** | **Estimated Concentration Ranges** |
| H₂O | 86.4 | ∼0.0 | 0-5% |
| Glycerin | 5.81 | 46.48 | 10-60% |
| Acrylamide | 5.74 | 45.92 | 10-60% |
| Bis-acrylamide | 0.07 | 0.56 | 0.01-2% |
| Guar Gum | 0.57 | 4.56 | 1-5% |
| Na₂CO₃ | 0.18 | 1.44 | 0.1-3% |
| Isopropanol | 1.03 | ∼0.0 | NA |
| TEMED | 0.05 | 0.4 | 0.05-0.6% |
| Ammonium Persulfate | 0.08 | 0.64 | 0.05-1.0% |

| **Dip Step** | | | |
|---|---|---|---|
| HOOH | 5 grams (pre-foaming) | 0-6% | 0-6% |
| O₂ | NA | 1.4-6.0% | 0.5-20% |

The silver nanoparticle coating resulted in an overall silver concentration of approximately 2000ppm (about 0.2 weight percent). The coating was applied by dipping the foamed dressing in an organic solvent containing silver nanoparticles. In this example the sliver nanoparticle solvent was heptane which has the advantage of having a high vapor pressure which can be easily evaporated leaving behind only silver nanoparticles.

### Example 2 - Oxygen-Containing Foam With Silver Chloride Incorporated Into The Foam Matrix

A polymer matrix containing oxygen gas was produced as an exemplary wound treatment device to deliver oxygen and antimicrobial silver material. The device was a closed cell foam sheet with oxygen gas contained in the cells of the foam. The closed cell foam sheet was made in accordance with the process described in the Detailed Description above and U.S. Patent No. 7,160,553 issued January 9, 2007 to Gibbins et al. for "Matrix For Oxygen Delivery To Compromised Tissues" and U.S. Patent No. 8,679,523 issued Mary 25, 2014 to Gibbins et al. for "Oxygen-Delivery Closed Cell Foam Matrix For Wound Treatment". The ingredients and their concentrations are listed in Table 2 below.

In use, the wound treatment device would be placed on a wound where it would then release the oxygen into the wound fluid. The silver would also be released such that the combination of silver and oxygen would yield the synergistic benefit described in the previous section.

| **TABLE 2** | | | |
|---|---|---|---|
| **OXYGEN-CONTAINING FOAM WITH SILVER CHLORIDE INCORPORATED INTO THE FOAM MATRIX** | | | |
| **Ingredient** | **Pre-Drying Concentration (Weight Percent of Batch)** | **Post-Drying Concentration (Weight Percent of Batch)** | **Estimated Ranges** |
| H2O | 86.04 | ∼0.0 | 0-5% |
| Glycerin | 5.81 | 46.48 | 10-60% |
| Acrylamide | 5.74 | 45.92 | 10-60% |
| Bis-acrylamide | 0.07 | 0.56 | 0.01-2% |
| Guar Gum | 0.57 | 4.56 | 1-5% |
| Na2CO3 | 0.18 | 1.44 | 0.1-3% |
| Isopropanol | 1.03 | ∼0.0 | NA |
| Silver Nitrate | 0.021 | 0.168 | 0.001-0.5% |
| Sodium Chloride | 0.01 | 0.07 | 0-0.5% |
| TEMED | 0.05 | 0.4 | 0.05-0.6% |
| Ammonium Persulfate | 0.08 | 0.64 | 0.05-1.0% |

| **Dip Step** | | | |
|---|---|---|---|
| HOOH | 5 grams (pre-foaming) | 0-6% | 0-6% |
| O2 | NA | 1.4-6.0% | 0.5-20% |

The silver chloride was incorporated into the gel sheet by adding sodium chloride and silver nitrate as solutions into the pre-polymerized mixture. Once the two are combined, silver chloride precipitates and is present generally throughout the closed cell foam sheet. The addition of silver chloride into the polymer mix solution resulted in an overall silver concentration of approximately 1000ppm (about 0.1 weight percent). The raw stock had a very slight darkening due to discoloration by the silver chloride. It was observed that the closed cell foam sheet did not disintegrate even after soaking overnight in deionized H₂O. No residual HOOH was detected.

### Example 3 - Oxygen Enhanced Silver Efficacy

In this experiment samples containing silver nitrate were compared to samples containing both silver nitrate and oxygen gas in an antimicrobial challenge test to demonstrate the synergy between oxygen and silver. In the first sample 10 ml of 5% tryptic soy broth (TSB) was inoculated with approximately 10⁵ CFU of *Pseudomonas aeruginosa* (ATCC 9027) and then spiked with silver nitrate until the final concentration was 0.03 ppm silver (approximately 0.000003 weight percent). The second sample was prepared similarly to sample 1 with the silver nitrate except that the headspace of the test tube was flooded with 100% oxygen gas (O₂) and then sealed. The oxygen in the headspace diffused in to the TSB in order to simulate the effect of an oxygen-delivering wound dressing as described in Examples 1 and 2. Each test sample was then incubated at 37°C; the level of surviving organisms was tested after 3 hours and 6 hours for each. Sample 1, which contained only silver, showed a 2 log reduction in the number of CFU after 3 hours and a 3 log reduction in the number of CFU after 6 hours. Sample 2, the silver and oxygen samples, showed a 3.2 log reduction in the number of CFU after 3 hours and a 4.5 log reduction in the number of CFU after 6 hours.

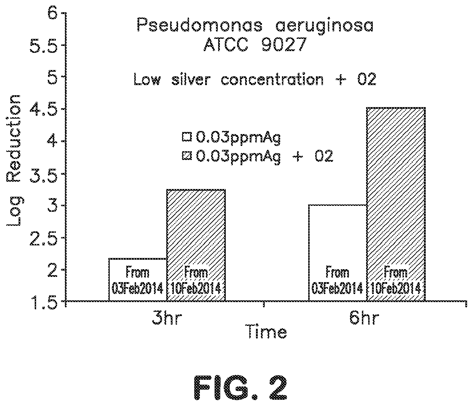

These results are illustrated in FIG. 2 which is an illustration of a graph depicting the log reduction in CFU on the y-axis and time (hours) on the x-axis. These results show a clear improvement in the log reduction for *Pseudomonas aeruginosa* when silver is used in conjunction with oxygen. For example, at 3 hours the silver + oxygen samples show more than a one full log reduction in the number of CFU (i.e., a reduction by a factor of 10) over the silver alone. At six hours the silver + oxygen samples show more than a 1.5 log reduction in the number of CFU (i.e., a reduction by a factor of 30) over silver alone.

Importantly, this example demonstrates the potentiating effect of gaseous oxygen on relatively low concentrations of antimicrobial silver. That is, the antimicrobial silver was present at 0.03 ppm silver (approximately 0.000003 weight percent).

### Testing Procedure for Dilute Media and Silver Concentrations

1. The challenge organism (*Pseudomonas aeruginosa* ATCC 9027) was prepared by culturing in Soybean-Casein digest broth (TSB) at 35°C for 18 to 24hrs to an approximate concentration of 1.8x10⁸ CFU per ml.
2. For the challenge tests: 0.01 ml of the stock culture was added into a 50 ml screw top test tube and then diluted to 10 ml with 5% TSB broth to a final concentration of 1.8x10⁵ CFU/ml.
3. Preparation of the challenge samples:
   a. Silver Challenge test: To a test tube prepared as described from step 2; 100 µl of 3 ppm AgNO₃ was added and vortexed to mix for a final concentration of 0.03 ppm AgNO₃.
   b. Silver + O₂ challenge test: To a test tube prepared as described from step 2; 100 µl of 3 ppm Ag+ (from AgNO₃) solution was added and vortexed to mix for a final concentration of 0.03 ppm AgNO₃. The headspace of the test tube was then purged with 100% USP oxygen gas for 2 minutes.
   c. The samples were then incubated at 37°C for both 3 and 6 hours.
   d. At the 3 and 6 hour time point the samples were plated at different dilutions (10⁻¹, 10⁻³, and 10⁻⁵) to enumerate the surviving colonies.
      i. The 3 hour samples were re-purged with O₂ gas and returned to the incubating oven for an additional 3 hours.
      ii. The media used for plating was Trypticase soy agar (TSA).
   e. The plates were incubated for 2 to 3 days before counting.
   f. The log reductions were based on the time zero plate count.

### Example 4 - Oxygen Enhanced Silver Efficacy

In a series of experiments four experimental samples were prepared to demonstrate the synergy between oxygen and silver. In the first sample,10 ml of 95% tryptic soy broth (TSB) was inoculated with approximately 10⁵ CFU of *Methicillin-resistant Staphylococcus aureus* - *MRSA* (Clinical isolate). The second sample was prepared similarly using another 10ml of 95% TSB that was inoculated to the same 10⁵ CFU but then spiked with silver nitrate until the final the final concentration was 3000 ppm silver (approximately 0.3 weight percent). The third sample was prepared similarly to Sample 2 with the silver nitrate except that the headspace of the test tube was flooded with 100% oxygen gas (O₂) and then sealed.). The fourth sample was prepared similarly to Sample 1 except that the headspace of the test tube was flooded with 100% oxygen gas (O₂) and then sealed. The oxygen in the headspace diffused in to the TSB in order to simulate the effect of an oxygen-delivering wound dressing as described in Examples 1 and 2. Each test sample was then incubated at 37°C; the level of surviving organisms was tested after 3 hours and 6 hours for each.

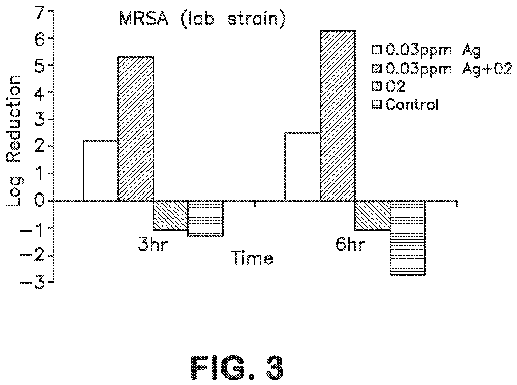

These results are illustrated in FIG. 3 which is an illustration of a graph depicting the log reduction in CFU on the y-axis and time (hours) on the x-axis. These results show a clear improvement in the log reduction for MRSA when silver is used in conjunction with oxygen (Sample 3). At 3 hours the silver samples show a 2.12 log reduction while the silver + oxygen samples show a 5.32 log reduction. After only 3 hours, the silver + oxygen samples provide more than a 3.2 log reduction in the number of CFU (i.e., a reduction by a factor of 1500) over the silver alone.

At six hours the silver + oxygen (Sample 3) samples show a 6.26 total log reduction versus a 2.5 log reduction from silver alone (Sample 2). After 6 hours, the silver + oxygen samples provide more than a 3.76 log reduction in the number of CFU (i.e., a reduction by a factor of 5700) over the silver alone. Controls were run in which contained oxygen gas alone (with no silver - Sample 4) and media only (no silver or O₂ - Sample 1). These are represented by the negative bars in the graph above which indicates that there was an increase (as expected) in the amount of colony forming units throughout the duration of the testing.

### Testing Procedure for Concentrated Media and Silver Samples

1. The challenge organism (MRSA Clinical isolate) was prepared by culturing in Soybean-Casein digest broth (TSB) at 35°C for 18 to 24hrs to an approximate concentration of 1.8x 10⁹ CFU per ml.
2. For the challenge tests: 0.01 ml of the stock culture was added into a 50 ml screw top test tube and then diluted to 10 ml with 95% TSB broth to a final concentration of 1.8x10⁶ CFU/ml.
3. Preparation of the challenge samples:
   a. Silver Challenge test: To a test tube prepared as described from step 2; 100 µl of 30% wt/wt AgNO₃ was added and vortexed to mix for a final concentration of 0.3% AgNO₃.
   b. Silver + O₂ challenge test: To a test tube prepared as described from step 2; 100 µl of 30% wt/wt AgNO₃ solution was added and vortexed to mix for a final concentration of 0.3% AgNO₃. The headspace of the test tube was then purged with 100% USP oxygen gas for 2 minutes.
   c. The samples were then incubated at 37°C for both 3 and 6 hours.
   d. At the 3 and 6 hour time point the samples were plated at different dilutions (10⁻¹, 10⁻³, and 10⁻⁵) to enumerate the surviving colonies.
      i. The 3 hour samples was re-purged with O₂ gas and returned to the incubating oven for an additional 3 hours.
      ii. The media used for plating was Trypticase soy agar (TSA). Sodium thioglycolate was added to the agar in order to neutralize the silver.
   e. The plates were incubated for 2 to 3 days before counting.
   f. The log reductions were based on the time zero plate count.

While the present invention has been described in connection with certain preferred embodiments it is to be understood that the subject matter encompassed by way of the present invention is not to be limited to those specific embodiments. On the contrary, it is intended for the subject matter of the invention to include all modifications as can be included within the scope of the following claims.

## Claims

1. A wound dressing comprising an oxygen delivery component and an antimicrobial silver material, such that when the wound dressing is used to treat a wound, oxygen is delivered in the presence of the antimicrobial silver material such that the antimicrobial silver material provides greater microbial kill than antimicrobial silver material alone, **characterised in that** the antimicrobial silver material is present at a concentration less than 0.5 weight percent.

2. The wound dressing of claim 1, for potentiating antimicrobial silver materials against pathogens comprising:
a) a formed biocompatible polymeric matrix, wherein the matrix includes closed cells and walls; and
b) gaseous oxygen, wherein the gaseous oxygen is contained in the closed cells;
wherein the antimicrobial silver material is present on at least a skin-contacting portion of the matrix; and
wherein oxygen is delivered from the closed cells in the presence of the antimicrobial silver material such that the antimicrobial silver material provides greater microbial kill than antimicrobial silver material alone.

3. The wound dressing of claim 2, wherein the biocompatible polymeric matrix comprises a polymer and a catalyst, and gaseous oxygen is produced when a peroxide is reacted with the catalyst so that dissolved oxygen is present in moisture in the walls.

4. The wound dressing of any one of the foregoing claims, for potentiating antimicrobial silver materials against pathogens, the device comprising:
a) a formed biocompatible matrix comprising: a polymer and a catalyst, wherein the matrix includes closed cells and walls;
b) gaseous oxygen, wherein the gaseous oxygen is contained in the closed cells; and
c) dissolved oxygen, wherein the dissolved oxygen is present in moisture in the walls;
wherein the antimicrobial silver material is present on a skin contacting portion of the matrix, wherein the gaseous oxygen and the dissolved oxygen are produced when a peroxide is reacted with the catalyst, such that when the device is used to treat a wound, oxygen is delivered from the closed cells in the presence of the antimicrobial silver material such that the antimicrobial silver material provides greater microbial kill than antimicrobial silver material alone.

5. The wound dressing of any one of the foregoing claims, wherein the antimicrobial silver material comprises a silver salt, a silver complex, elemental silver, or a combination thereof.

6. The wound dressing of claim 5, wherein the elemental silver is selected from silver powder and silver nanoparticles.

7. The wound dressing of any one of claims 2 to 6, wherein the catalyst comprises sodium carbonate, cupric chloride, ferric chloride, manganese oxide, sodium iodide, lactoperoxidase, catalase, or a combination thereof, and wherein the peroxide comprises hydrogen peroxide, ammonium peroxide, sodium peroxide, or a combination thereof.

8. The wound dressing of any one of claims 2 to 7, wherein the biocompatible matrix further comprises a stranded configuration.

9. A non-therapeutic method of potentiating antimicrobial silver material against pathogens comprising:
providing antimicrobial silver nanoparticles to a surface or article to be treated, **characterised in that** the antimicrobial silver is provided at a concentration less than 0.5 weight percent; and
providing oxygen in the presence of the antimicrobial silver so the antimicrobial silver material provides greater microbial kill than antimicrobial silver material alone.

10. The method of claim 9, wherein the oxygen is provided by providing a formed matrix that includes closed cells and walls, wherein gaseous oxygen is contained in the closed cells at a concentration greater than atmospheric concentration, preferably wherein the antimicrobial silver nanoparticles are provided present on a portion of the matrix.

11. The method of claim 9 or 10, wherein the article or surface to be treated is an inanimate article or surface.

12. The method of any of claims 9-11, wherein the oxygen is provided to the article at greater than atmospheric concentration.

13. The wound dressing of any of claims 1-8, wherein the antimicrobial silver material is antimicrobial silver nanoparticles.

14. The wound dressing of any of claims 1-8 and 13 for use with oxygen at greater than atmospheric concentration in treating or preventing microbial infection, enhancing wound healing, or treating a bacterial infection.

## Patentansprüche

1. Wundverband, der eine Sauerstofflieferkomponente und ein antimikrobielles Silbermaterial derart umfasst, dass, wenn der Wundverband verwendet wird, um eine Wunde zu behandeln, Sauerstoff in der Gegenwart des antimikrobiellen Silbermaterials derart geliefert wird, dass das antimikrobielle Silbermetall größere mikrobielle Abtötung bereitstellt als das antimikrobielle Silbermaterial allein, **dadurch gekennzeichnet, dass** das antimikrobielle Silbermaterial in einer Konzentration von weniger als 0,5 Gew.-% vorliegt.

2. Wundverband nach Anspruch 1 zum Potenzieren antimikrobiellen Silbermaterials gegen Pathogene, der Folgendes umfasst:
a) eine geformte biokompatible polymerische Matrix, wobei die Matrix geschlossene Zellen und Wände beinhaltet; und
b) gasförmigen Sauerstoff, wobei der gasförmige Sauerstoff in den geschlossenen Zellen enthalten ist;
wobei das antimikrobielle Silbermaterial auf mindestens einem Abschnitt der Matrix, der die Haut kontaktiert, vorliegt; und
wobei Sauerstoff von den geschlossenen Zellen in der Gegenwart des antimikrobiellen Silbermaterials derart geliefert wird, dass das antimikrobielle Silbermaterial größere mikrobielle Abtötung bereitstellt als antimikrobielles Silbermetall allein.

3. Wundverband nach Anspruch 2, wobei die biokompatible polymerische Matrix ein Polymer und einen Katalysator umfasst, und gasförmiger Sauerstoff erzeugt wird, wenn ein Peroxid mit dem Katalysator reagiert, so dass gelöster Sauerstoff in Feuchtigkeit in den Wänden vorliegt.

4. Wundverband nach einem der vorstehenden Ansprüche, zum Potenzieren antimikrobieller Silbermaterialien gegen Pathogene, wobei die Vorrichtung Folgendes umfasst:
a) eine geformte biokompatible Matrix, die Folgendes umfasst: ein Polymer und einen Katalysator, wobei die Matrix geschlossene Zellen und Wände beinhaltet;
b) gasförmigen Sauerstoff, wobei der gasförmige Sauerstoff in den geschlossenen Zellen enthalten ist; und
c) gelösten Sauerstoff, wobei der gelöste Sauerstoff in Feuchtigkeit in den Wänden vorliegt;
wobei das antimikrobielle Silbermaterial auf einem Abschnitt der Matrix, der die Haut kontaktiert, vorliegt, wobei der gasförmige Sauerstoff und der gelöste Sauerstoff erzeugt werden, wenn ein Peroxid mit dem Katalysator reagiert, so dass, wenn die Vorrichtung verwendet wird, um eine Wunde zu behandeln, Sauerstoff von den geschlossenen Zellen in der Gegenwart des antimikrobiellen Silbermaterials derart geliefert wird, dass das antimikrobielle Silbermaterial größere mikrobielle Abtötung bereitstellt als antimikrobielles Silbermaterial allein.

5. Wundverband nach einem der vorstehenden Ansprüche, wobei das antimikrobielle Silbermaterial ein Silbersalz, einen Silberkomplex, elementares Silber oder eine Kombination davon umfasst.

6. Wundverband nach Anspruch 5, wobei das elementare Silber aus Silberpulver und Silbernanopartikeln ausgewählt ist.

7. Wundverband nach einem der Ansprüche 2 bis 6, wobei der Katalysator Natriumcarbonat, Kupferchlorid, Eisenchlorid, Manganoxid, Natriumjodid, Lactoperoxidase, Katalase oder eine Kombination davon umfasst, und wobei das Peroxid Wasserstoffperoxid, Ammoniumperoxid, Natriumperoxid oder eine Kombination davon umfasst.

8. Wundverband nach einem der Ansprüche 2 bis 7, wobei die biokompatible Matrix weiter eine verseilte Konfiguration umfasst.

9. Nicht-therapeutisches Verfahren zum Potenzieren antimikrobiellen Silbermaterials gegen Pathogene, das Folgendes umfasst:
Bereitstellen antimikrobieller Silbernanopartikel auf einer Oberfläche oder einem Artikel, die/der zu behandeln ist, **dadurch gekennzeichnet, dass** das antimikrobielle Silber mit einer Konzentration von weniger als 0,5 Gew.-% bereitgestellt wird; und
Bereitstellen von Sauerstoff in der Gegenwart antimikrobiellen Silbers derart, dass das antimikrobielle Silbermaterial größere mikrobielle Abtötung bereitstellt als antimikrobielles Silbermaterial allein.

10. Verfahren nach Anspruch 9, wobei der Sauerstoff bereitgestellt wird, indem eine geformte Matrix bereitgestellt wird, die geschlossene Zellen und Wände beinhaltet, wobei gasförmiger Sauerstoff in den geschlossenen Zellen mit einer Konzentration enthalten ist, die größer ist als atmosphärische Konzentration, wobei bevorzugt die antimikrobiellen Silbernanopartikel auf einem Abschnitt der Matrix vorliegen.

11. Verfahren nach Anspruch 9 oder 10, wobei der Artikel oder die Oberfläche, der/die zu behandeln ist, ein unbelebter Artikel oder eine unbelebte Oberfläche ist.

12. Verfahren nach einem der Ansprüche 9-11, wobei der Sauerstoff zu dem Artikel mit einer Konzentration, die größer ist als atmosphärische Konzentration, bereitgestellt wird.

13. Wundverband nach einem der Ansprüche 1-8, wobei das antimikrobielle Silbermaterial aus antimikrobiellen Silbernanopartikeln besteht.

14. Wundverband nach einem der Ansprüche 1- 8 und 13 für die Verwendung mit Sauerstoff mit einer größeren Konzentration als die atmosphärische Konzentration, beim Behandeln oder Verhindern mikrobieller Infektion, Verbessern von Wundheilung oder Behandeln einer bakteriellen Infektion.

## Revendications

1. Pansement pour plaie comprenant un composant de distribution d'oxygène et un matériau d'argent antimicrobien, de telle sorte que, lorsque le pansement pour plaie est utilisé pour traiter une plaie, de l'oxygène soit distribué en présence du matériau d'argent antimicrobien de telle sorte que le matériau d'argent antimicrobien confère une plus grande élimination microbienne que le matériau d'argent antimicrobien seul, **caractérisé en ce que** le matériau d'argent antimicrobien est présent à une concentration inférieure à 0,5 pour cent en poids.

2. Pansement pour plaie selon la revendication 1, pour potentialiser des matériaux d'argent antimicrobien contre des agents pathogènes comprenant :
a) une matrice polymère biocompatible formée, dans lequel la matrice inclut des cellules fermées et des parois ; et
b) de l'oxygène gazeux, dans lequel l'oxygène gazeux est contenu dans les cellules fermées ;
dans lequel le matériau d'argent antimicrobien est présent sur au moins une partie en contact avec la peau de la matrice ; et
dans lequel de l'oxygène est distribué depuis les cellules fermées en présence du matériau d'argent antimicrobien de telle sorte le matériau d'argent antimicrobien confère une plus grande élimination microbienne que le matériau d'argent antimicrobien seul.

3. Pansement pour plaie selon la revendication 2, dans lequel la matrice polymère biocompatible comprend un polymère et un catalyseur, et de l'oxygène gazeux est produit lorsqu'un peroxyde est mis en réaction avec le catalyseur de telle sorte que de l'oxygène dissous soit présent dans l'humidité dans les parois.

4. Pansement pour plaie selon l'une quelconque des revendications précédentes, pour potentialiser des matériaux d'argent antimicrobien contre des agents pathogènes, le dispositif comprenant :
a) une matrice biocompatible formée comprenant : un polymère et un catalyseur, dans lequel la matrice inclut des cellules fermées et des parois ;
b) de l'oxygène gazeux, dans lequel l'oxygène gazeux est contenu dans les cellules fermées ; et
c) de l'oxygène dissous, dans lequel l'oxygène dissous est présent dans l'humidité dans les parois ;
dans lequel le matériau d'argent antimicrobien est présent sur une partie en contact avec la peau de la matrice, dans lequel l'oxygène gazeux et l'oxygène dissous sont produits lorsqu'un peroxyde est mis en réaction avec le catalyseur, de telle sorte que, lorsque le dispositif est utilisé pour traiter une plaie, de l'oxygène soit distribué à partir des cellules fermées en présence du matériau d'argent antimicrobien de telle sorte que le matériau d'argent antimicrobien confère une plus grande élimination microbienne que le matériau d'argent antimicrobien seul.

5. Pansement pour plaie selon l'une quelconque des revendications précédentes, dans lequel le matériau d'argent antimicrobien comprend un sel d'argent, un complexe d'argent, de l'argent élémentaire ou une combinaison de ces derniers.

6. Pansement pour plaie selon la revendication 5, dans lequel l'argent élémentaire est sélectionné parmi une poudre d'argent et des nanoparticules d'argent.

7. Pansement pour plaie selon l'une quelconque des revendications 2 à 6, dans lequel le catalyseur comprend du carbonate de sodium, du chlorure de cuivre, du chlorure de fer, de l'oxyde de manganèse, de l'iodure de sodium, de la lactoperoxydase, de la catalase ou une combinaison de ces derniers, et dans lequel le peroxyde comprend du peroxyde d'hydrogène, du peroxyde d'ammonium, du peroxyde de sodium ou une combinaison de ces derniers.

8. Pansement pour plaie selon l'une quelconque des revendications 2 à 7, dans lequel la matrice biocompatible comprend en outre une configuration torsadée.

9. Procédé non thérapeutique de potentialisation d'un matériau d'argent antimicrobien contre des agents pathogènes comprenant les étapes consistant à :
fournir des nanoparticules d'argent antimicrobien à une surface ou à un article à traiter, **caractérisé en ce que** l'argent antimicrobien est fourni à une concentration inférieure à 0,5 pour cent en poids ; et
fournir de l'oxygène en présence de l'argent antimicrobien de telle sorte que le matériau d'argent antimicrobien confère une plus grande élimination microbienne que le matériau d'argent antimicrobien seul.

10. Procédé selon la revendication 9, dans lequel l'oxygène est fourni en fournissant une matrice formée qui inclut des cellules fermées et des parois, dans lequel de l'oxygène gazeux est contenu dans les cellules fermées à une concentration supérieure à la concentration atmosphérique, de préférence dans lequel les nanoparticules d'argent antimicrobien sont fournies en étant présentes sur une partie de la matrice.

11. Procédé selon la revendication 9 ou 10, dans lequel l'article ou la surface à traiter est un article ou une surface inanimé(e).

12. Procédé selon l'une quelconque des revendications 9 à 11, dans lequel l'oxygène est fourni à l'article à une concentration supérieure à la concentration atmosphérique.

13. Pansement pour plaie selon l'une quelconque des revendications 1 à 8, dans lequel le matériau d'argent antimicrobien est des nanoparticules d'argent antimicrobien.

14. Pansement pour plaie selon l'une quelconque des revendications 1 à 8 et 13 destiné à être utilisé avec de l'oxygène à une concentration supérieure à la concentration atmosphérique lors du traitement ou de la prévention d'une infection microbienne, de l'amélioration de la cicatrisation d'une plaie ou du traitement d'une infection bactérienne.
